# EUROPEAN PATENT APPLICATION

(11) **EP 2 907 879 A2**
(43) Date of publication of application: **19.08.2015**
(21) Application number: 15158646.8
(22) Date of filing: 29.04.2010
(51) Int. Cl.: C12Q 1/68, C12N 15/113, A61K 31/7105

(54) **Means and methods for counteracting, preventing and/or determining heart failure, or a risk of heart failure**

(30) Priority: 29.04.2009 WO PCT/NL2009/050237; 26.08.2009 EP 09168764
(62) Divisional of application: 10769995.1
(71) Applicant: Academisch Medisch Centrum bij de Universiteit van Amsterdam, 1105 AZ Amsterdam (NL)
(72) Inventor: Creemers, Esther Elisa Johanna Maria, 1058 TS Amsterdam (NL); Pinto, Yigal-Martin, 1186 BR Amstelveen (NL); Tijsen, Anke Johanna Marina, 3607 PB Maarssen (NL)
(74) Representative: DeltaPatents B.V.

(57) **Abstract**

The present invention relates to a method for diagnosing and/or prognosing heart failure in a subject the method comprising the step of determining the level of at least one genetic marker in a body fluid sample obtained from said subject, wherein said at least one genetic marker is an miRNA. The invention further relates to a method for treating or preventing heart failure in a subject the method comprising the step of decreasing within said subject the expression, amount, and/or activity of at least one miRNA, or decreasing within said subject the interaction of at least one miRNA with its mRNA target or increasing within said subject the expression, amount, and/or activity of the mRNA target of at least one miRNA.

## Description

### FIELD OF THE INVENTION

The invention relates to the fields of biology and medicine. More in particular, the present invention relates to miRNAs for use in diagnosis and therapy of heart failure.

### BACKGROUND OF THE INVENTION

MicroRNAs (miRNAs) are small RNA molecules encoded in the genomes of plants and animals. They are present within introns of protein-coding genes, in polycistronic transcripts encoding multiple miRNAs and in individual miRNA genes. These highly conserved RNAs with a length of approximately 21-23 nucleotides usually regulate the expression of genes by binding to the 3'-untranslated regions (3'-UTRs) of specific mRNAs. Each miRNA is thought to regulate multiple genes, and since hundreds of miRNA genes are predicted to be present in higher eukaryotes the potential regulatory circuitry afforded by miRNA is enormous. Several research groups have provided evidence that miRNAs act as key regulators of processes as diverse as early development, cell proliferation and cell death, apoptosis and fat metabolism, and cell differentiation. There is speculation that in higher eukaryotes, the role of miRNAs in regulating gene expression could be as important as that of transcription factors. Over 540 human miRNAs have been validated to date; however, computer models suggest there may be thousands more. It is believed that up to 30% of human genes are regulated by miRNAs.

The genes that encode miRNAs are transcribed from DNA by RNA polymerase II but not translated into protein. The primary transcripts, which generally have a length of several kilobases, are called pri-miRNAs. Pri-miRNAs are processed in the cell nucleus to shorter, 70-100 nucleotide stem-loop structures known as pre-miRNAs. This processing is performed in animals by the RNase III endonuclease Drosha. Pre-miRNAs are subsequently transported into the cytoplasm, where they are processed to double-stranded miRNAs with a length of 21-23 nucleotides by a second RNase III endonuclease, DICER. One strand of the miRNA duplex is subsequently incorporated into the RNA-induced silencing complex (RISC). As part of the RISC, gene expression of a target gene is counteracted by inhibiting translation and/or by cleaving mRNA. The mature miRNAs are partially complementary to one or more mRNA molecules. If miRNA and mRNA are expressed in the same cell, they can hybridize in a sequence-specific manner, thereby preventing translation of the mRNA into protein, thus importantly regulating specific protein levels.

The level of interest and confidence in miRNAs as possible clinical players can be demonstrated by the fact that various miRNA-based biotechnology companies have been started up recently.

Several miRNAs have been linked to cancer and heart disease. Expression analysis studies reveal perturbed miRNA expression in tumors compared to normal tissues. MicroRNAs are deregulated in breast, lung, and colon cancer, and upregulated in Burkitt's and other human B-cell lymphomas. As a consequence, human miRNAs are likely to be highly useful as biomarkers, especially for future cancer diagnostics, and are rapidly emerging as attractive targets for disease intervention. In addition to their link with cancer, microRNAs play an important role in the control of diverse aspects of cardiac function and dysfunction, including myocyte growth, integrity of the ventricular wall, contractility, gene expression, and maintenance of cardiac rhythm. The misexpression of miRNAs has been shown to be necessary and sufficient for multiple forms of heart disease.

It was recently described that miRNAs are also present in blood. Several miRNAs could be detected at relatively high levels in serum, plasma, platelets, erythrocytes, as well as the nucleated blood cells. Aberrant expression profiles of miRNAs have been described in blood of subjects with sickle cell anaemia or in prostate cancer.

Although it is certain that genetic factors are associated with the predisposition to develop heart failure, the identification of such genetic factors has been problematic given the complex nature of the disorder.

Methods for diagnosing acute coronary syndromes, including heart failure, have *inter alia* been described in WO/2002/083913, WO2002/089657 and WO2006/120152. All these methods use the level of brain natriuretic peptide (BNP) as a marker for the acute coronary syndrome in for instance blood or urine. However, this polypeptide is secreted by the ventricles of the heart in response to excessive stretching of heart muscle cells, and is therefore a physiological marker reflecting the heart condition. There is a need for genetic markers, which more accurately reflect the genetic predisposition of a subject of developing heart failure. Such markers also allow diagnosis more early.

Hence, the availability of genetic markers that are predictive for developing heart failure is highly desirable. At present, no such markers exist.

### SUMMARY OF THE INVENTION

It has now been found that miRNA blood profiles are altered in heart disease. Without whishing to be bound by any theory, it is believed that cells, including cardiac myocytes, excrete these miRNAs via exosomes into the blood and that the excretion pattern is changed in disease.

Thus, the present inventors have shown that miRNA profiling in blood can be used as a novel tool for diagnostic or biomarker approaches in heart failure.

It is an object of the present invention to provide means and methods for treating, preventing or determining (a risk of) heart failure.

Accordingly, the present invention provides in a first aspect a method for treating or preventing heart failure in a subject the method comprising the step of:
- decreasing within said subject the expression, amount, and/or activity of at least one miRNA; or
- decreasing within said subject the interaction of at least one miRNA with its mRNA target; or
- increasing within said subject the expression, amount, and/or activity of the mRNA target of at least one miRNA,
wherein said at least one miRNA is selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR18b*, miR-1254, miR-622, HS_202.1, miR-302d and solexa-3927-221.

In another aspect the present invention provides a method for treating or preventing heart failure in a subject the method comprising the step of:
- increasing within said subject the expression, amount, and/or activity of at least one miRNA; or
- increasing within said subject the interaction of at least one miRNA with its mRNA target; or
- decreasing within said subject the expression, amount, and/or activity of the mRNA target of at least one miRNA,
wherein said at least one miRNA is selected from the group consisting of miR-191, miR-30e, miR-744, miR-142-3p and solexa-2952-306.

In preferred embodiments of the above aspect, said step of increasing the expression, amount and/or activity or the interaction of said miRNA comprises administering to said subject said miRNA as functional miRNA, as precursor microRNA (pre-miRNA), or as microRNA primary transcript (pri-miRNA).

In another aspect the present invention provides a miRNA selected from the group consisting of miR-191, miR-30e, miR-744, miR-142-3p and solexa-2952-306, or a precursor microRNA (pre-miRNA) or a microRNA primary transcript thereof, for use as a medicament, preferably for use in the treatment or prevention of heart failure.

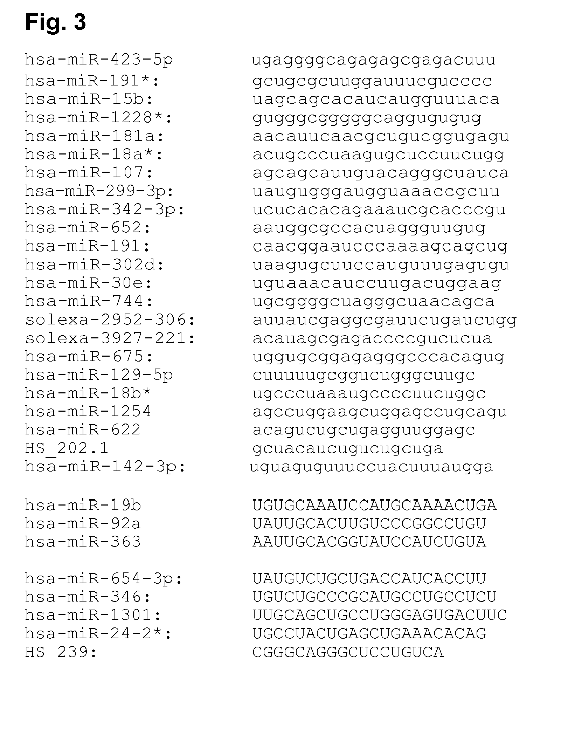

In yet another aspect, the present invention provides a compound, preferably a nucleic acid, capable of specifically binding to and thereby inhibiting the activity and/or function of at least one miRNA selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d and solexa-3927-221, and precursor microRNAs (pre-miRNA) or microRNA primary transcripts thereof for use as a medicament, preferably for use in the treatment or prevention of heart failure. The term "function" herein refers in particular to the capacity of the said miRNA to inhibit translation of the target mRNA. The nucleic acid is therefore preferably capable of inhibiting the hybridization in a sequence-specific manner between the miRNA and its mRNA target. The term "specifically binding to" in the context of a nucleic acid as defined herein refers in particular to a nucleic acid that is capable of hybridizing under stringent conditions to the said miRNA. In a preferred embodiment, the said nucleic acid has a sequence that is the complement of the sequence of the miRNA as provided in Figure 3.

In another aspect the present invention provides a vector comprising a nucleic acid sequence encoding a nucleic acid capable of specifically binding to and thereby inhibiting the activity and/or function of at least one miRNA selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d and solexa-3927-221, and precursor microRNAs (pre-miRNA) or microRNA primary transcripts thereof or a miRNA selected from the group consisting of miR-191, miR-30e, miR-744, miR-142-3p and solexa-2952-306, and precursor microRNAs (pre-miRNA) and microRNA primary transcripts thereof, and capable of expressing said nucleic acid or (pre or pri) miRNA, for use as a medicament, preferably for use in the treatment or prevention of heart failure.

In yet another aspect the present invention provides a cell comprising a vector according to the invention as described above, for use as a medicament, preferably for use in the treatment or prevention of heart failure.

In still another aspect the present invention provides a pharmaceutical composition for treating or preventing heart failure in a subject comprising:
- a gene according to the invention as described above or the expression product thereof, a (pre- or pri-) miRNA according to the invention as described above, a vector according to the invention as described above, or a cell according to the invention as described above, and
- a pharmaceutically acceptable carrier, diluent or excipient.

In still a further aspect the present invention provides a method for diagnosing a subject as suffering from heart failure or being at risk of developing heart failure, the method comprising:
a) determining the level of a genetic marker in a sample obtained from said subject, wherein said genetic marker is a miRNA selected from the group consisting of miR-191*, miR-15b, miR-1228*, miR-181a, miR-18a*, miR-107, miR-299-3p, miR-342-3p, miR-652, miR-675, miR-191, miR-302d, miR-30e, miR-744, miR-142-3p, solexa-2952-306, and solexa-3927-221, and precursor microRNAs (pre-miRNA) or microRNA primary transcripts thereof;
b) diagnosing whether the subject suffers from heart failure or is at risk of developing heart failure based on the level of a genetic marker determined in step a).

In a further embodiment, the invention provides a method for diagnosing a subject as suffering from heart failure or being at risk of developing heart failure, the method comprising a) determining the level of at least one genetic marker in a sample obtained from said subject, wherein said at least one genetic marker is a miRNA selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221, and precursor microRNAs (pre-miRNA) or microRNA primary transcripts thereof; and b) diagnosing whether the subject suffers from heart failure or is at risk of developing heart failure based on the level of said at least one genetic marker determined in step a). In a preferred embodiment, the said at least one genetic marker comprises at least two miRNAs, more preferred at least three miRNAs selected from miRNA selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221 and precursor microRNAs (pre-miRNA) or microRNA primary transcripts thereof. In a further preferred embodiment, the at least two miRNAs are selected from the group consisting of miR-423-5p and miR-191*, miR-423-5p and miR-15b, miR-423-5p and miR-1228*, miR-423-5p and miR-181a, miR-423-5p and miR-18a*, miR-423-5p and miR-107, miR-423-5p and miR-299-3p, miR-423-5p and miR-342-3p, miR-423-5p and miR-652, miR-423-5p and miR-675, miR-423-5p and miR-129-5p, miR-423-5p and miR-18b*, miR-423-5p and miR-1254, miR-423-5p and miR-622, miR-423-5p and HS_202.1, miR-423-5p and miR-191, miR-423-5p and miR-302d, miR-423-5p and miR-30e, miR-423-5p and miR-744, miR-423-5p and miR-142-3p, miR-423-5p and solexa-2952-306, or miR-423-5p and solexa-3927-221.

In still a further aspect the present invention provides a method for diagnosing a subject as suffering from heart failure or being at risk of developing heart failure, the method comprising:
a) determining the level of a genetic marker in a sample obtained from said subject, wherein said genetic marker is a miRNA selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221, and precursor microRNAs (pre-miRNA) or microRNA primary transcripts thereof;
b) diagnosing whether the subject suffers from heart failure or is at risk of developing heart failure based on the level of a genetic marker determined in step a).

In a preferred embodiment of said method, said step b) takes into account that said subject suffers from heart failure or is at risk of developing heart failure when:
- the expression, amount, and/or activity of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d and/or solexa-3927-221, or precursor microRNAs (pre-miRNA) or microRNA primary transcripts thereof, is upregulated relative to a control subject not suffering from or not being at risk of developing heart failure; and/or
- the expression, amount, and/or activity of miR-191, miR-30e, miR-744, miR-142-3p, and/or solexa-2952-306, or precursor microRNAs (pre-miRNA) or microRNA primary transcripts thereof, is downregulated relative to a control subject not suffering from or not being at risk of developing heart failure.

The invention further provides a method for treating or preventing heart failure in a subject, the method comprising diagnosing whether the subject suffers from heart failure or is at risk of developing heart failure based on the level of at least one genetic marker according the invention; and treating the subject when the subject suffers from heart failure or is at risk of developing heart failure.

In another aspect the present invention provides a kit of parts for diagnosing a subject as suffering from heart failure or being at risk of developing heart failure comprising:
- at least one compound capable of specifically binding to at least one miRNA selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221, and precursor microRNAs (pre-miRNA) or microRNA primary transcripts thereof; and
- instructions for use of said compound for diagnosing a subject as suffering from heart failure or being at risk of developing heart failure according to a method of the present invention.

In another aspect the present invention provides a method for determining whether a candidate compound is capable of treating or preventing heart failure in a human subject, the method comprising:
- contacting said candidate compound with a human tissue cell, preferably a cardiac tissue cell, and
- determining whether said candidate compound is capable of modulating the expression, amount and/or activity of any one of miR-423-5p, miR-191*, miR-15b, miR-1228*, miR-181a, miR-18a*, miR-107, miR-299-3p, miR-342-3p, miR-652, miR-191, miR-302d, miR-30e, miR-744, miR-675, miR-129-5p, miR-18b*, miR-1254, miR-622, HS_202.1, miR-142-3p, solexa-2952-306, and solexa-3927-221 in said tissue cell. Preferably, said compound affects the expression of all miRNAs into a direction no longer associated with (a risk of) heart failure.

In a preferred method for determining whether a candidate compound is capable of treating or preventing heart failure in a human subject, the method comprises:
- contacting said candidate compound with a tissue cell, preferably a human tissue cell, more preferably a cardiac tissue cell, and
- determining whether said candidate compound is capable of decreasing the expression, amount and/or activity of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d solexa-3927-221 and/or increasing the expression of miR-191, miR-30e, miR-744, miR-142-3p, or solexa-2952-306 in said tissue cell.

Also part of the invention, in all aspects and embodiments, are the miRNAs miR-654-3p, miR-346, miR-1301, miR-24-2 and HS_239, which were found to be differentially expressed in plasma of HF patients, when compared to plasma of healthy controls.

The aspect of the invention pertaining to the screening of candidate therapeutic compounds may also be performed in a model system, for instance an animal model, which exhibits specific miRNA expression patterns associated with (an increased risk of) heart failure as indicated herein. Thus a further aspect of the invention is a (animal, including human) model system that exhibits a miRNA expression profile associated with (an increased risk of) heart failure as indicated herein. Such model systems may be used in methods for screening for candidate therapeutic agents comprising the step of exposing (cells of) said model system to or administering to said model system a candidate compound and determining whether said candidate compound is capable of inducing a change in said miRNA expression profile that is no longer associated with (an increased risk of) heart failure, in particular capable of increasing the expression, amount and/or activity of miR-191, miR-30e, miR-744, miR-142-3p or solexa-2952-306 in (cells of) said model system and/or lowering the expression, amount and/or activity of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d or solexa-3927-221 in (cells of) said model system.

### BRIEF DESCRIPTION OF THE DRAWINGS

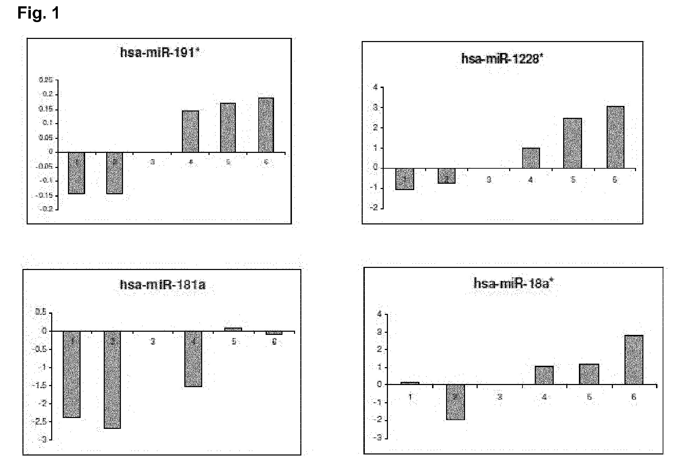

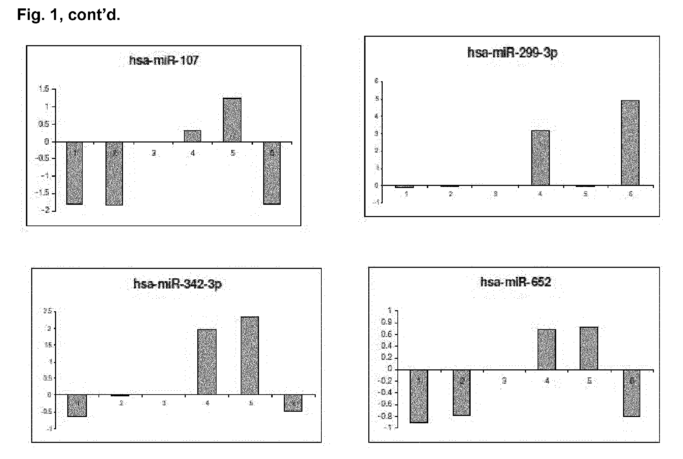

Figure 1 shows miRNAs enriched in plasma of heart failure (HF) patients. Plasma samples. 1) healthy person 1; 2) healthy person 2; 4) HF patient 1; 5) HF patient 2; 6) HF patient 3.

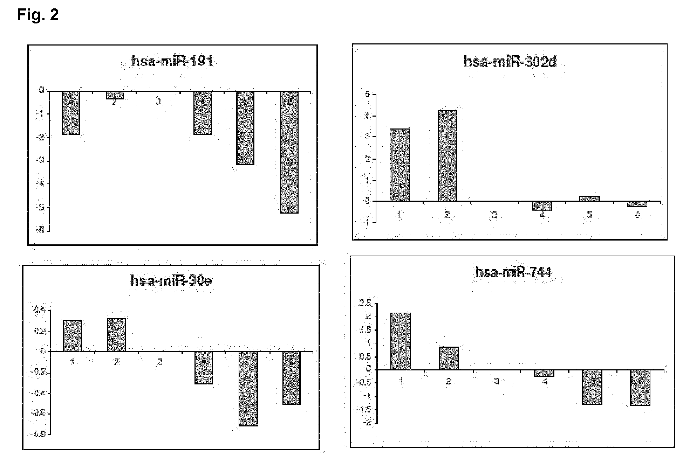

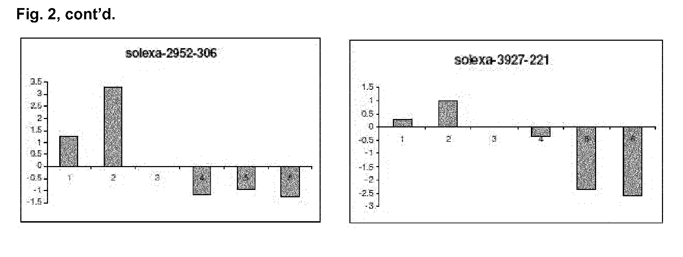

Figure 2 shows miRNAs down-regulated in plasma of heart failure patients. Plasma samples: 1) healthy person 1; 2) healthy person 2; 4) HF patient 1; 5) HF patient 2; 6) HF patient 3.
Figure 3 shows the sequences (5'-3') of the miRNAs as discussed herein.

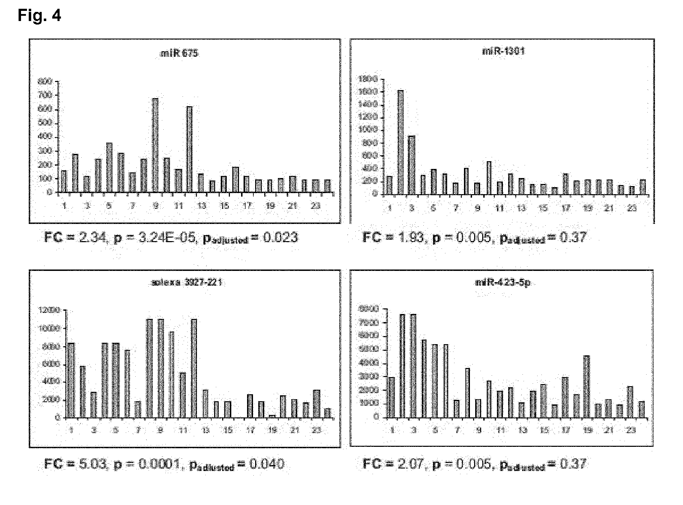

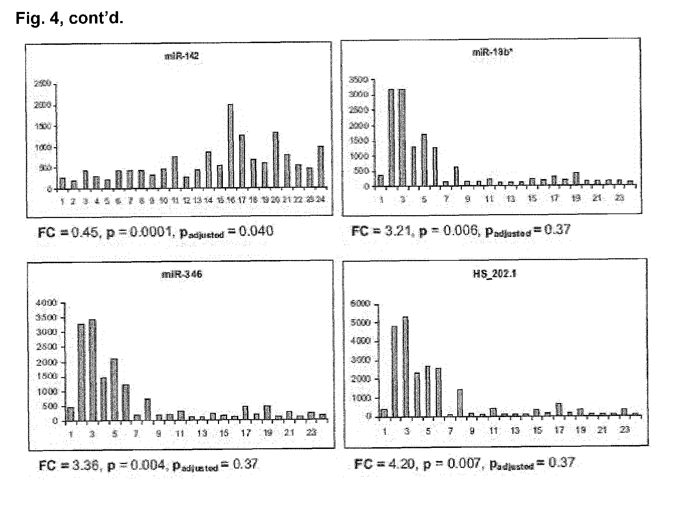

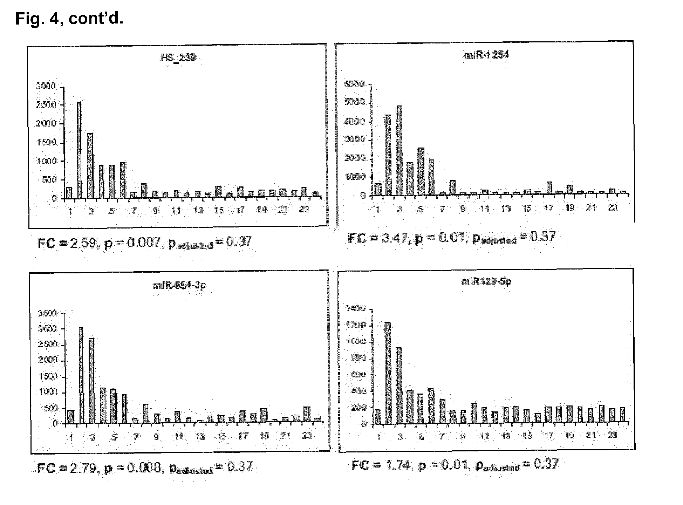

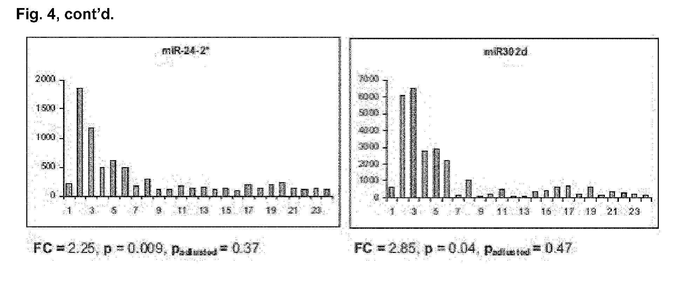

Figure 4 shows the expression of the miRNAs as discussed herein as measured on a DNA chip array, wherein per miRNA 24 measurements are shown. The first 12 are measurements from patient samples and the next 12 (nos. 13-24) are controls. FC = fold change; p = p value; and p_{adjusted} is the p value corrected for multiple testing. The results were confirmed by QPCR.

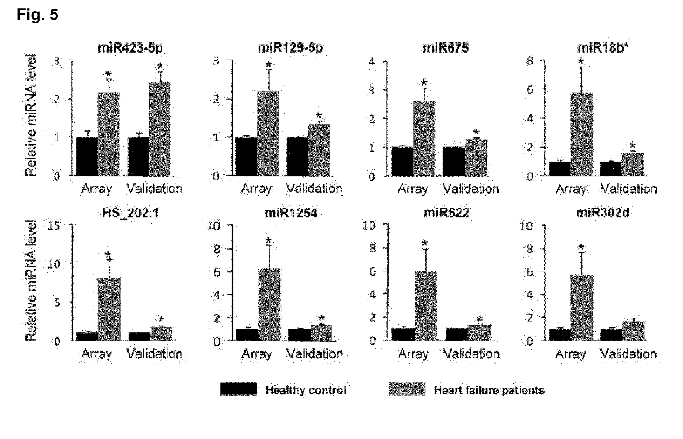

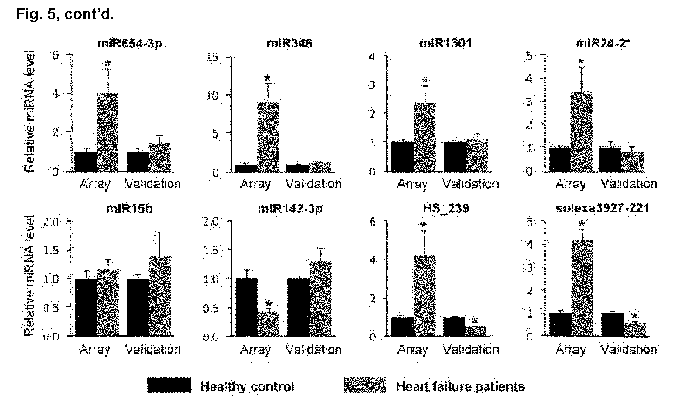

Figure 5 shows expression profiles of 16 candidate miRNAs in plasma of HF patients and control subjects. Left 2 bars of each panel show miRNA levels determined by miRNA array in 12 healthy controls and 12 HF patients. Right 2 bars show levels of the same miRNA now validated by real-time PCR in separate subjects (30 HF cases and 39 healthy controls). Data are presented as means ±SEM. *P<0.05 compared to healthy controls.

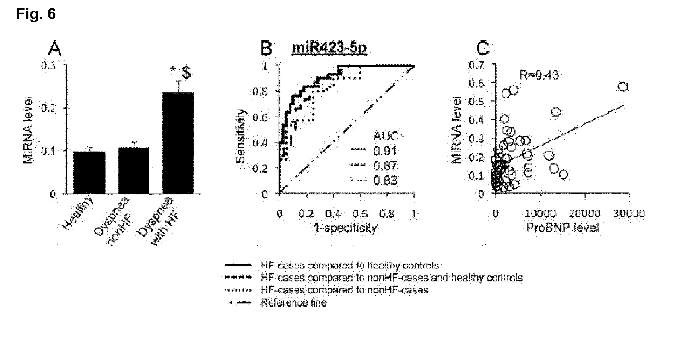

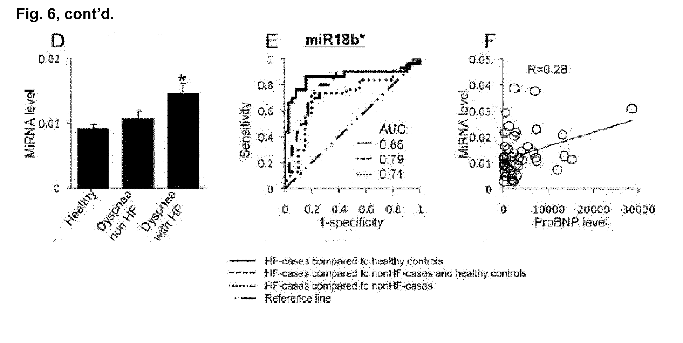

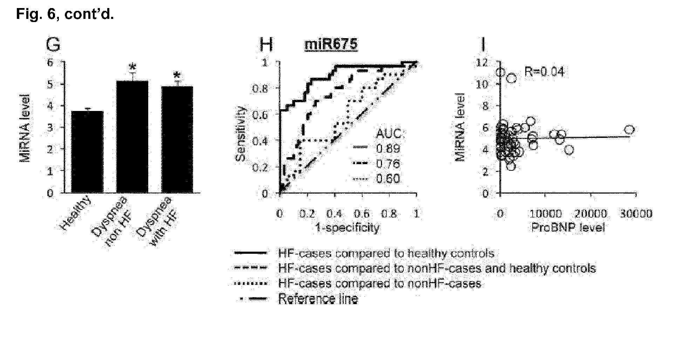

Figure 6 shows diagnostic accuracy of miRNAs. A, D, and G show miRNA levels in healthy controls, non-HF cases, and HF cases. Data are shown as mean ± SEM. *P<0.05 compared to healthy controls; $: P<0.05 compared to non-HF cases. B, E, and H show ROC curves and AUC regarding diagnostic power to distinguish HF from non-HF cases. C, F, and I show Spearman correlation between proBNP and miRNA, which is significant for miR-423-5p (P=0.002) and borderline significant for miR-18b* (P=0.049). Omission of the single outlier did not affect correlation of miR423-5p but reduced that of miR-18b*.

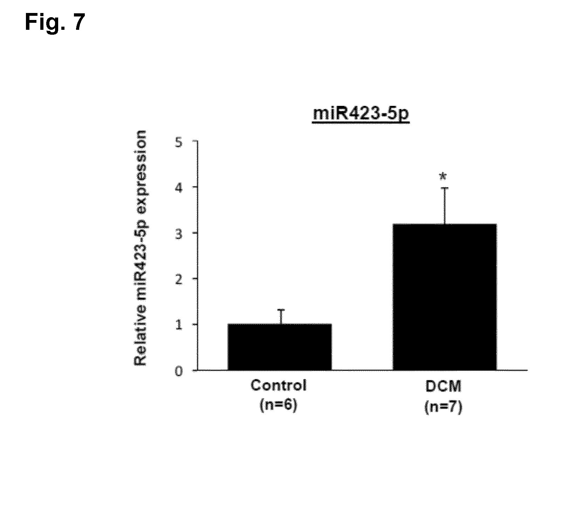

Figure 7 shows miRNA expression in postmortem myocardial tissue from subjects who had died due to dilated cardiomyopathy (DCM) compared to subjects who had died due to noncardiac causes (control). Real-time PCR for miR-423-5p was normalized for expression of U6. * denotes p<0.05 compared to controls

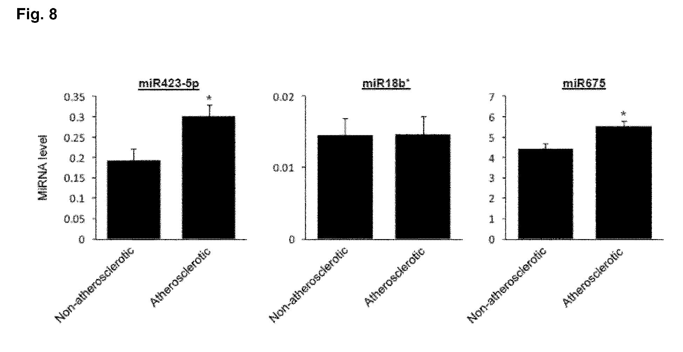

Figure 8 shows levels of circulating miRNAs in subjects with atherosclerotic versus non-atherosclerotic forms of heart failure. To compare levels of circulating miRNAs according to the underlying cause of heart failure, we distinguished atherosclerotic forms of HF and non-atherosclerotic forms of HF. It is important to stress here that in this study we excluded patients who showed actual signs of myocardial ischemia, so that active ischemia was excluded as a cause of HF. 12 patients out of 30 had a history of chronic extensive coronary atherosclerotic disease leading to HF. These subjects with atherosclerotic disease showed significantly higher circulating levels of miR-423-5p and miR-675, but not of miR-18b*, compared to the patients with non-atherosclerotic HF (n=18). *: p<0.05 compared to nonatherosclerotic forms.

### DESCRIPTION OF THE PREFERRED EMBODIMENTS

The term "heart failure" or "cardiac failure" refers to the pathophysiologic state in which the heart, via an abnormality of cardiac function (detectable or not), fails to pump blood at a rate commensurate with the requirements of the metabolizing tissues and/or pumps only from an abnormally elevated diastolic filling pressure. Heart failure may be caused by myocardial failure but may also occur in the presence of near-normal cardiac function under conditions of high demand. Heart failure always causes circulatory failure, but the converse is not necessarily the case because various noncardiac conditions (eg, hypovolemic shock, septic shock) can produce circulatory failure in the presence of normal, modestly impaired, or even supranormal cardiac function. The term "heart failure" thus includes systolic and diastolic heart failure, acute heart failure and chronic heart failure, irrespective of the cause of heart failure, and irrespective of whether or not there is a genetic component associated with the heart failure and/or the cause of heart failure.

As used herein, "nucleic acid" includes reference to a deoxyribonucleotide or ribonucleotide polymer in either single-or double-stranded form, and unless otherwise limited, encompasses known analogues having the essential nature of natural nucleotides in that they hybridize to single-stranded nucleic acids in a manner similar to naturally occurring nucleotides (e. g., peptide nucleic acids).

The term "isolated", as in "isolated cell", refers to material, such as a cell, which is substantially or essentially free from components that normally accompany or interact with it as found in its naturally occurring environment. An isolated cell may be a cell culture obtained by culturing a cell that has been separated from a tissue in which it normally resides and that is no longer integrated in the tissue of the organism from which it is derived.

"Nucleotides" are referred to by their commonly accepted single-letter codes following IUPAC nomenclature: A (Adenine), C (Cytosine), T (Thymine), G (Guanine), U (Uracil), W (A or T), R (A or G), K (G or T), Y (C or T), S (C or G), M (A or C), B (C, G or T), H (A, C, or T), D (A, G, or T), V (A, C, or G), N (A, C, G, or T).

A "coding" or "encoding" sequence is the part of a gene that codes for the amino acid sequence of a protein, or for a functional RNA such as a tRNA or rRNA.

The term "gene", as used herein refers to a DNA sequence including but not limited to a DNA sequence that can be transcribed into mRNA which can be translated into polypeptide chains, transcribed into rRNA or tRNA or serve as recognition sites for enzymes and other proteins involved in DNA replication, transcription and regulation. The term refers to any DNA sequence comprising several operably linked DNA fragments such as a promoter region, a 5' untranslated region (the 5' UTR), a coding region (which may or may not code for a protein), and an untranslated 3' region (3' UTR) comprising a polyadenylation site. Typically, the 5'UTR, the coding region and the 3'UTR are transcribed into an RNA of which, in the case of a protein encoding gene, the coding region is translated into a protein. The gene usually comprises introns and exons. A gene may include additional DNA fragments such as, for example, introns.

"Sample" is used in its broadest sense as containing nucleic acids. A sample may comprise a bodily fluid such as blood or urine; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint, buccal cells, skin, or hair; and the like.

A preferred sample for detection of a miRNA according to the invention is a body fluid selected from blood and urine. A most preferred sample is a blood sample. A blood sample may comprise a whole blood sample, or a sample that is obtained by centrifugation and/or filtration such as, for example, plasma, serum, platelets, red blood cell, white blood cells, as is known to the skilled person. A blood sample may be obtained by venepuncture, arteripuncture and/or capillary puncture such as, for example, a finger prick. The sample, preferably a blood sample, may be collected in a tube comprising an anticoagulant such as a heparin tube or an EDTA-tube, as is known to the skilled person.

The present invention relates to the diagnostic, prognostic and therapeutic use of miRNAs indicated herein as hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221. The nucleotide sequence of these miRNAs is provided in Figure 3.

MiRNAs counteract the expression of a specific gene product by annealing to the mRNA encoded by the gene that encodes the proteinaceous gene product. Thus, miRNA has the ability to translationally repress a target mRNA thereby regulating a target mRNA functionally.

Expression of the gene products encoded by these mRNAs in a cell is thus influenced by influencing the expression, amount and/or activity of at least one miRNA selected from the group consisting of hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221, or a functional part or derivative thereof, within said cell, and/or by influencing interaction of the target mRNA with at least one miRNA selected from the group consisting of hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221, or a functional part or derivative thereof, within said cell. A use of at least one miRNA selected from the group consisting of hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221 or a functional part or derivative thereof, or use of a compound capable of influencing the expression, amount and/or activity of at least one miRNA selected from the group consisting of hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221 in a cell, or use of a compound capable of influencing interaction of the target mRNA with at least one miRNA selected from the group consisting of hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221, for influencing expression of a specific gene product encoded by the miRNA's target mRNA in said cell is therefore also provided. In one embodiment, expression of the specific gene product is influenced in vitro, for instance in a cell culture.

The miRNAs follow a standard nomenclature system, as is known to the skilled person. An uncapitalized "mir-" refers to a pre-miRNA, while a capitalized "miR-" refers to a mature form. MiRNAs with nearly identical sequences are annotated with an additional lower case letter. For example, miR-123a is closely related to miR-123b. MiRNAs that are 100% identical but are encoded at different places in the genome are indicated with additional dash-number suffix: miR-123-1 and miR-123-2 are identical but are produced from different pre-miRNAs. Species of origin is designated with a three-letter prefix, e.g., hsa-miR-123 would be from human (Homo sapiens) and oar-miR-123 would be a sheep (Ovis aries) miRNA. When relative expression levels are known, an asterisk following the name indicates an miRNA expressed at low levels relative to the miRNA in the opposite arm of a hairpin. For example, miR-123 and miR-123* would share a pre-miRNA hairpin, but relatively more miR-123 would be found in the cell. The suffices 5p and 3p indicate if a miRNA is derived from the 5'arm or the 3'arm of the pre-miRNA respectively. These suffices are used when both miRNAs are expressed at equal levels. For example miR423-5p and miR423-3p would share a pre-miRNA hairpin. According to the present invention, miRNAs as mentioned herein are capable of counteracting expression of specific gene products. As used herein, the term "miRNA" encompasses any isoform of the said miRNA and all members of the said miRNA family are capable of counteracting expression of the specific gene products. The term "miRNA" thus includes precursors such as pri-miRNA and pre-miRNA, star-sequences such as miR-123 and miR-123*, family members such as miR-17 and miR-18; and cluster members such as miR-92a, miR-92a-2*, and miR-363.

Thus, the term miR-423-5p includes the isoforms pre-mir423 and miR423-3p; the term miR-129-5p includes the isoforms pre-mir129-1, pre-mir129-2, miR-129* and miR-129-3p; the term miR-18b* includes the isoforms pre-mir18b, miR-18b, pre-mir17, miR-17, miR-17*, pre-mir18a, miR-18a, miR-18a*, pre-mir20a, miR-20a, pre-mir20b, miR-20b, miR-20b*, pre-mir93, miR-93, miR-93*, pre-mir106a, miR-106a, miR-106a*, pre-mir106b, miR-106b, miR-106b*, pre-mir19b-2, miR-19b, miR-19b-2*, pre-mir92a-2, miR-92a, miR-92a-2*, pre-mir363, miR-363, and miR-363*; the term miR-1254 includes the isoforms pre-mir1254; the term miR-622 includes the isoforms pre-mir622; the term HS_202.1 includes the isoforms pre-mir221, miR-221 and miR-221*.

Hence, as used herein, the term "miRNA" encompasses any miRNA member. Although hsa-miR-423-5p, hsa-miR-191*, has-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, has-miR-302d, hsa-miR-30e, hsa-miR-744, hsa-miR-142-3p, solexa-2952-306, and solexa-3927-221 are known in the art, their association with heart failure and their effect on heart failure was unknown before the present invention.

Now that the invention has provided the insight that the above-mentioned miRNAs are associated with heart failure, it has become possible to diagnose, prevent and treat heart failure. The association between the miRNAs described herein and heart failure is through specific gene products which expression is prevented or regulated by the miRNAs. Counteracting the expression of certain of these specific gene products will result in a lower amount of these specific gene products, which in turn will diminish the onset, progression and/or effects of heart failure. This is particularly true for gene products whose expression is regulated by miRNAs that are upregulated during heart failure. In addition, stimulating the expression of certain of these specific gene products, for instance by preventing the expression of the miRNAs, will result in a higher amount of other of these specific gene products, which may also diminish the onset, progression and/or effects of heart failure. This is particularly true for gene products whose expression is regulated by miRNAs that are downregulated during heart failure. These specific gene products are therefore indicated as important mediators of heart failure, by yet unknown mechanisms. According to the present invention, several of the miRNAs selected from hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1, hsa-miR-191, hsa-miR-302d, hsa-miR-30e, hsa-miR-744, hsa-miR-142-3p, solexa-2952-306, solexa-3927-221 are capable of counteracting heart failure, by counteracting the expression of specific gene products.

One embodiment therefore provides a method for counteracting, treating, diminishing, delaying and/or preventing heart failure, in a subject, the method comprising:
- increasing the expression, amount and/or activity of an miRNA selected from the group consisting of hsa-miR-191, hsa-miR-30e, hsa-miR-744, hsa-miR-142-3p, solexa-2952-306 or a functional part or derivative thereof, within said subject, and/or
- increasing the interaction of the specific target mRNA with a miRNA selected from the group consisting of hsa-miR-191, hsa-miR-30e, hsa-miR-744, hsa-miR-142-3p, solexa-2952-306, or with a functional part or derivative thereof, within said subject. As described herein before, said miRNA comprises any member of the said miRNA family, and/or
- decreasing the expression, amount and/or activity of a miRNA selected from the group consisting of hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, has-mir-675, hsa-miR-129-5p, hsa-miR-18b*, hsa-miR-1254, hsa-miR-622, HS_202.1, miR-302d and/or solexa-3927-221 or a functional part or derivative thereof, within said subject, and/or
- decreasing the interaction of the specific target mRNA with a miRNA selected from the group consisting of from hsa-miR-423-5p, hsa-miR-191*, hsa-miR-15b, hsa-miR-1228*, hsa-miR-181a, hsa-miR-18a*, hsa-miR-107, hsa-miR-299-3p, hsa-miR-342-3p, hsa-miR-652, hsa-miR-675, hsa-miR-129-5p, hsa-miR-18b*, hsa-miR-1254, hsa-miR-622, HS_202.1,and/or solexa-3927-221, or with a functional part or derivative thereof, within said subject. As described herein before, said miRNA comprises any member of the said miRNA family.

In one embodiment, said subject has been diagnosed with heart failure. In another embodiment, however, said subject has an increased risk of heart failure, for instance because said subject is already suffering from injury or disease. In an alternative embodiment, a method according to the invention is performed in order to generally prevent heart failure, in an unaffected subject.

Methods for diagnosing heart failure are known in the art. A subject is for instance diagnosed with heart failure, by determining whether mechanical stiffness is increased, whether normal functions, such as for instance electrical properties of the heart, are decreased, whether ECM proteins are accumulated excessively and/or whether levels of specific gene products are above a certain threshold level. An increased risk of heart failure is often present in a subject when the subject is suffering from an injury or disorder.

Methods of the invention are suitable for diagnostic and therapeutic use for heart failure. In one preferred embodiment a method according to the invention is used in order to counteract, treat, diminish, delay and/or prevent cardiac failure. Further provided is therefore a method for treating and/or preventing heart failure in a subject, the method comprising:
- increasing the expression, amount and/or activity of the miRNAs that are downregulated in heart failure as identified herein, and/or decreasing the expression, amount and/or activity of the miRNAs that are upregulated in heart failure as identified herein, within a subject in need of such treatment and/or prevention. This may for instance be accomplished by increasing the interaction between the target mRNAs of these miRNAs and/or by decreasing the interaction between the target mRNAs of these miRNAs, for the downregulated and upregulated miRNAs respectively.

In one preferred embodiment, said subject has been diagnosed with cardiac failure, or with a risk of cardiac failure. In an alternative embodiment, said method is performed in order to generally prevent cardiac failure, in an unaffected subject.

It is possible to increase the expression, amount and/or activity of a miRNA downregulated in heart failure as indicated herein directly, for instance by administering an activating compound capable of increasing the expression and/or activity of the miRNA. It is also possible to increase the expression, amount and/or activity of the miRNA indirectly, for instance by decreasing the expression, amount and/or activity of an inhibitor of the miRNA. The opposite is true for decreasing the expression, amount and/or activity of a miRNA upregulated in heart failure as indicated herein.

In a preferred embodiment, the amount of the miRNA is increased by administering the pri-miRNA and/or the pre-miRNA and/or the miRNA, or a functional part or derivative thereof, to said subject. Administration of any of these compounds, or any combination of these compounds, results in (increased) counteraction of target mRNA and, thus, counteraction and/or prevention of heart failure. The pri-miRNA is the primary transcript of the miRNA, having a length of several kilobases, which is obtained after transcription of the genomic miRNA sequence by RNA polymerase II. Pre-miRNA is the shorter, 70-100 nucleotide stem-loop structure which is obtained after processing of the pri-miRNA by the ribonuclease Drosha. The sequences of the miRNAs as indicated herein are depicted in Figure 3.

A functional part of the pri-miRNA or pre-miRNA or miRNA is defined herein as a nucleic acid sequence with a length of at least 7 nucleotides which is shorter then the pri-miRNA or pre-miRNA or (mature) miRNA, respectively, and which is capable of binding a target mRNA gene (the gene encoding the mRNA to which the miRNA binds) and/or target mRNA and counteracting expression of the protein from the target mRNA or the gene. Said functional part is preferably capable of binding the same 3' UTR region of target mRNA which is capable of being bound by the miRNA. Said functional part preferably comprises a sequence of the seed region of the miRNA. A seed region of the miRNA is present in the 5' region of the naturally occurring miRNA. Said seed region is particularly involved in target recognition. The sequence of the seed region of the miRNA can be found by the skilled artisan without difficulty or inventive skill by performing binding experiments between the miRNA and the mRNA. A functional part of the pri-miRNA or pre-miRNA or miRNA is thus preferably a nucleic acid sequence with a length of at least 7 nucleotides, comprising the seed region sequence. However, slight modifications of said seed region are allowed, as long as the capability of binding a target mRNA gene or the target mRNA is maintained. A functional part of the pri-miRNA or pre-miRNA or miRNA thus also comprises a nucleic acid sequence with a length of at least 7 nucleotides, comprising a sequence which has at least 72%, more preferably at least 80%, more preferably at least 86%, most preferably at least 90% sequence identity with the sequence of the seed region of the miRNAs as indicated herein.

A functional derivative of the pri-miRNA or pre-miRNA or miRNA is defined herein as a molecule which comprises a sequence which has 70% or more, but less than 100%, sequence identity with the pri-miRNA or pre-miRNA or miRNA and which has at least one same property in common irrespective of quantitative considerations. Said functional equivalent is capable of binding a target mRNA gene and/or target mRNA and counteracting expression of the protein encoded by the said gene, albeit not necessarily to the same extent as the pri-miRNA or pre-miRNA or miRNA. Such functional equivalent for instance comprises:
- a polynucleotide wherein one or more nucleotides are added to the native sequence of the pri-miRNA or pre-miRNA or miRNA;
- a pri-miRNA or pre-miRNA or miRNA molecule wherein between 1-40 % of the nucleotides are substituted by one or more other nucleotides, as long as the seed region sequence retains at least 72% sequence identity with the miRNA sequence as indicated in Figure 3; and/or
- a pri-miRNA or pre-miRNA or miRNA derivative wherein at least one nucleotide has been modified so that the resulting product has a non-naturally occurring nucleotide. Preferably, a functional equivalent of the pri-miRNA or pre-miRNA or miRNA has a nucleotide sequence having at least about 75% sequence identity with the pri-miRNA or pre-miRNA or miRNA, preferably at least about 80%, more preferably at least about 85%, more preferably at least about 90%, most preferably at least about 95%. The higher the sequence identity, the more closely said functional equivalent resembles the pri-miRNA or pre-miRNA or miRNA.

A preferred example of a functional equivalent of the pri-miRNA or pre-miRNA or miRNA is a vector comprising at least the seed region of the miRNA, or a vector comprising a sequence which has at least 72%, preferably at least 80%, more preferably at least 86%, most preferably at least 90% sequence identity with the seed region sequence.

The term "% sequence identity" is defined herein as the percentage of nucleotides in a nucleic acid sequence that is identical with the nucleotides in a nucleic acid sequence of interest, after aligning the sequences and optionally introducing gaps, if necessary, to achieve the maximum percent sequence identity. Methods and computer programs for alignments are well known in the art. As used herein, the terms "nucleic acid sequence" and "nucleotides" also encompass non-natural molecules based on and/or derived from nucleic acid sequences, such as for instance artificially modified nucleic acid sequences, peptide nucleic acids, as well as nucleic acid sequences comprising at least one modified nucleotide and/or non-natural nucleotide such as for instance inosine.

Methods for introducing polynucleotides into a cell are known in the art. Methods for introducing nucleic acid for instance comprise calcium phosphate transfection, DEAE-Dextran, electroporation or liposome-mediated transfection. Alternatively, direct injection of the polynucleotide is employed. Preferably however, a nucleic acid sequence is introduced into a cell by a vector, preferably a viral vector. Said vector preferably comprises a retroviral, adenoviral, adeno-associated viral (AAV), or lentiviral vector. In one embodiment an AAV9 vector is used.

Various terms are known in the art which refer to introduction of nucleic acid into a cell by a vector. Examples of such terms are "transduction", "transfection" and "transformation". Techniques for generating a vector with a nucleic acid sequence and for introducing said vector into a cell are known in the art.

Preferably, the pri-miRNA or pre-miRNA or miRNA or a functional part or derivative thereof is used which is able to be introduced into a mammalian cell in vivo. Non-limiting examples of methods according to the invention are the coupling of said nucleic acid sequence to cell-penetrating peptides, to microcarriers or to nanocarriers, or the use of liposomes containing said nucleic acid sequence. Preferably, said inhibitor is targeted to heart muscle cells, for instance by using artificial HDL-like particles bound to said inhibitor, enhancing delivery to the myocardium.

According to the present invention, heart failure, is particularly well counteracted and/or prevented by administering the pri-miRNA or pre-miRNA or miRNA that is downregulated or underexpressed in said subject, or a functional part or derivative thereof, to a subject suffering from, or at risk of suffering from, heart failure. Heart failure, is also counteracted and/or prevented by administering a compound capable of increasing or restoring the expression, amount and/or activity of a miRNA that is downregulated or underexpressed in said subject, or by administering a compound capable of increasing interaction of the target mRNA with the miRNA that is downregulated or underexpressed in said subject. The invention therefore further provides pri-miRNA and/or pre-miRNA and/or miRNA that is downregulated or underexpressed in said subject, or a functional part or derivative thereof, or a compound capable of increasing or restoring the expression, amount and/or activity of miRNA that is downregulated or underexpressed in heart failure as indicated herein in a cell, or a compound capable of increasing interaction of the target mRNA with the miRNA that is downregulated or underexpressed in heart failure as indicated herein, for use in counteracting, treating, diminishing, delaying and/or preventing heart failure. The opposite is true for the miRNA that is upregulated or overexpressed in heart failure as indicated herein.

The above-mentioned compounds, or any combination thereof, are thus particularly suitable for the preparation of a medicament or prophylactic agent against heart failure. Further provided is therefore a use of the pri-miRNA and/or pre-miRNA and/or miRNA as indicated herein, or a functional part or derivative thereof, or a use of a compound capable of increasing or restoring the expression, amount and/or activity of the miRNA in a cell, or a use of a compound capable of increasing interaction of the target mRNA with the said miRNA, for the preparation of a medicament or prophylactic agent against heart failure. Said prophylactic agent is particularly suitable for subjects with an increased risk of heart failure, as well as for subjects already suffering from heart failure. However, said prophylactic agent is also suitable for generally preventing heart failure, in unaffected subjects.

The above can also be accomplished by increasing or decreasing the amount or activity of the protein product encoded by the target mRNA of the miRNAs as identified herein. Administration of any of the above mentioned compounds, or any combination thereof, to a subject is particularly suitable for counteracting and/or preventing heart failure, in said subject. In one embodiment, any of the above mentioned compounds, or any combination thereof, is administered to a subject who has been diagnosed with heart failure. In another embodiment, any of the above mentioned compounds, or any combination thereof, is administered to a subject with an increased risk of heart failure, for instance a subject who is already suffering from injury or disease. However, said compounds, or any combination thereof, are also suitable for generally preventing heart failure, in unaffected subjects.

As already described, methods for establishing the presence or risk of heart failure, include examination of heart functioning. It has now been found that a method can be provided which includes measurement of the accumulation of (pri/pre) miRNA levels or the levels of the proteins encoded by the target mRNAs as identified herein. However, now that the present invention has disclosed the presence of a connection between a group of miRNAs and heart failure, additional methods have become available. According to the invention, the presence or risk of heart failure, is established by determining whether the amount of the pri-miRNA and/or pre-miRNA and/or miRNAs associated with heart failure as identified herein in a sample of a subject is above or below a certain reference value. In one embodiment said reference value represents the amount of pri-miRNA and/or pre-miRNA and/or miRNA selected from the group consisting of miR-191, miR-30e, miR-744, miR-142-3p and solexa-2952-306 in a healthy subject or healthy population. If the amount of the said pri-miRNA and/or pre-miRNA and/or miRNA in a sample of a subject is significantly below the reference value, the expression of the target mRNA is not sufficiently counteracted. As a result, expression of the protein encoded by the target mRNA will be too high and the subject is suffering from, or at risk of suffering from, heart failure. In such case, a therapy according to the present invention is recommended.

On the other hand, if the amount of the pri-miRNA and/or pre-miRNA and/or miRNA of the miRNAs selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d and/or solexa-3927-221 in a sample of a subject is significantly above the reference value, target mRNA expression (or the translation of the target mRNA into protein) is severely counteracted. In this case, the subject is also diagnosed as suffering from, or at risk of suffering from, heart failure.

The amount of the pri-miRNA and/or pre-miRNA and/or miRNAs associated with heart failure may be determined after isolation of RNA from a sample, preferably a blood sample or urine sample. The isolation of RNA is preferably performed in the presence of a strong denaturant such as GITC, LiCl, SDS and/or phenol in order to inactivate RNase, if present. Methods for isolating RNA are known in the art, including the use of commercial RNA isolation kits such as, for example, mirVana PARIS kit (Ambion) en Trizol LS (Invitrogen). However, a sample can be processed for detection of miRNA sequences without prior isolation of RNA, for example by isolating vesicles such as microvesicles from a sample.

Methods for detection of a miRNA in a sample are known to the skilled person and include Northern blotting, the use of micro- en macroarrays, in situ hybridization, single molecule detection in liquid phase, massively parallel sequencing and quantitative polymerase chain reaction (Q-PCR). The RNA in a sample is preferably amplified prior to detection of miRNA sequences. Methods for amplification of RNA sequences are known in the art and include reverse-transcriptase polymerase chain reaction (RT-PCR) and Nucleic Acid Sequence Based Amplification (NASBA). A preferred amplification method comprises quantitative amplification of the RNA sequences by, for example, the use of SYBR GREEN (Roche, Basel, Switzerland) or fluorescently labeled Taqman probes (Applied Biosystems, Foster City, USA).

Primers for reverse transcriptase-mediated cDNA synthesis may be provided by the provision of a shared sequence to all miRNA sequences such as, for example, a poly(A)-tail by ligation or through action of a Terminal Transferase, followed by annealing of an adapter-oligo(dT) primer. Further methods comprise the use of a stem-loop primer, and/or the use of a miRNA-specific primer. The quantitative amplification of the RNA sequences, preferably by real-time PCR, preferably comprises a universal primer and a miRNA-specific primer.

The primers used for detection, cDNA synthesis and/or amplification preferably comprise RNA nucleotides, DNA nucleotides or modified nucleotides such as Locked Nucleic Acid (LNA) nucleotides, Peptide Nucleic Acid (PNA) nucleotides, and/or 2'-O-alkyl modifications, 2'-fluoro modifications, 4'-thio modifications, a phosphorotioate linkage, a morpholino linkage, a phosphonocarboxylate linkage. In a preferred embodiment, the length of a primer, preferably a miRNA-specific primer, is identical to the length of the specific miRNA. In a further preferred embodiment, the length of the miRNA-specific primer is shorter than the length of the miRNA, for example 14 nucleotides, 15 nucleotides, 16 nucleotides, 17 nucleotides, 18 nucleotides, 19 nucleotides, 20 nucleotides, 21 nucleotides, 22 nucleotides, or 23 nucleotides, depending on the length of the specific miRNA. The sequence of a primer, preferably a miRNA-specific primer, preferably comprises one or two mismatches compared to the sequence of the miRNA or the adapter sequence that is added to the miRNA, more preferably is identical to the sequence of the miRNA.

Methods and means for detection of one or more miRNAs in a sample are preferably provided as a kit. Said kit preferably comprises a set of primers, preferably at least one specific set of primers, enzymes such as a RNA-dependent DNA polymerase and/or a DNA-dependent DNA polymerase, and at least one buffer for performing the reaction or reactions. Said kit may be provided as dried material, for example after lyophilisation, or as a liquid.

If the amount of the pri-miRNA and/or pre-miRNA and/or miRNA in a sample of a subject is essentially the same as the reference value, target mRNA expression is sufficiently counteracted. In this case, the subject is not diagnosed as suffering from, or at risk of suffering from, heart failure.

Of course, other kinds of reference values than those of healthy subjects can be used. For instance, a reference value can be used that represents the amount of the pri-miRNA and/or pre-miRNA and/or miRNA in a subject or population suffering from heart failure. In this case, a sample with an amount of the pri-miRNA and/or pre-miRNA and/or miRNA which is essentially the same as said reference value indicates (a risk of) heart failure. A sample wherein said value is much smaller or larger indicates health.

The invention therefore provides a method for determining whether a subject is suffering from heart failure, or whether a subject has an increased risk of developing heart failure, the method comprising:
- obtaining a sample from said subject;
- determining in said sample the amount of the pri-miRNA and/or pre-miRNA and/or miRNA indicated as associated with heart failure herein;
- comparing said amount with a reference amount; and
- determining from said comparison whether or not the subject is suffering from heart failure, or is having an increased risk of developing heart failure.

The amount of the pri-miRNA and/or pre-miRNA and/or miRNA in a sample of a subject is preferably established using a binding compound. At least part of a sample is preferably contacted with such binding compound (optionally after previous processing of the sample), where after unbound components are preferably washed away and bound compounds are preferably visualized and quantified. One embodiment thus provides a method according to the invention for determining whether a subject is suffering from heart failure, or whether a subject has an increased risk of developing heart failure, the method further comprising contacting at least part of said sample with at least one compound capable of specifically binding the pri-miRNA and/or pre-miRNA and/or miRNA. Of course, if only a part of said sample is used, a part is used which contains microRNAs so that a suitable test is carried out.

The sample is preferably a blood or plasma or urine sample.

The invention furthermore provides a kit of parts for carrying out a method according to the present invention. Further provided is thus a kit of parts comprising:
- at least one compound capable of specifically binding the pri-miRNA and/or pre-miRNA and/or miRNA indicated herein as associated with heart failure; and
- instructions for use of said compound for determining whether a subject is suffering from heart failure.

The invention furthermore provides a kit of parts for carrying out a therapy according to the present invention. With such kit of part, target mRNA expression is typically counteracted via miRNA. A kit of parts according to the invention comprises:
- the pri-miRNA and/or pre-miRNA and/or miRNA indicated herein as downregulated or underexpressed in heart failure, or a functional part or derivative thereof, and/or
- a compound capable of increasing or restoring the expression, amount and/or activity of the pri-miRNA and/or pre-miRNA and/or miRNA indicated herein as downregulated or underexpressed in heart failure in a cell, and/or
- a compound capable of increasing interaction of the target mRNA with the pri-miRNA and/or pre-miRNA and/or miRNA indicated herein as downregulated or underexpressed in heart failure, and
- instructions for use of said compound(s) for counteracting, treating, diminishing, delaying and/or preventing heart failure, in a subject in need thereof. Said pri-miRNA and/or pre-miRNA and/or miRNA indicated herein as downregulated or underexpressed in heart failure, or a functional part or derivative thereof is preferably present in a vector. It is also possible to use a vector comprising a nucleic acid sequence encoding any of the above-mentioned compounds. Said vector preferably comprises a viral vector, most preferably a retroviral, adenoviral, adeno-associated viral or lentiviral vector. In one embodiment an AAV9 vector is used.

Further provided is therefore a vector comprising:
- the pri-miRNA and/or pre-miRNA and/or miRNA indicated herein as downregulated or underexpressed in heart failure, or a functional part or derivative thereof, and/or
- a nucleic acid sequence encoding a compound capable of increasing or restoring the expression, amount and/or activity of the miRNA indicated herein as downregulated or underexpressed in heart failure in a cell, and/or
- a nucleic acid sequence encoding a compound capable of increasing interaction of the target mRNA with the miRNA indicated herein as downregulated or underexpressed in heart failure.

In one preferred embodiment, said vector comprises:
- one or more of miR-191, miR-30e, miR-744, miR-142-3p and solexa-2952-306, or a pre- or pri-miRNA thereof, or a functional part or derivative thereof, and/or
- a nucleic acid sequence encoding a compound capable of increasing or restoring the expression, amount and/or activity of one or more of miR-191, miR-30e, miR-744, miR-142-3p and solexa-2952-306 in a cell, and/or
- a nucleic acid sequence encoding a compound capable of increasing interaction of the target mRNA of one or more of miR-191, miR-30e, miR-744, miR-142-3p and solexa-2952-306, with miR-191, miR-30e, miR-744, miR-142 and solexa-2952-306. Such a vector is particularly suitable for counteracting heart failure.

A vector according to the invention preferably comprises a retroviral, adenoviral, adeno-associated viral or lentiviral vector. In one embodiment, said vector comprises an AAV9 vector.

Any of the above mentioned nucleic acid sequences of a vector according to the invention are preferably operably linked to a promoter, so that expression of said nucleic acid will occur after transfection. Said promoter is preferably suitable for expression in a mammalian cell. In a particularly preferred embodiment, a vector according to the invention is suitable for expression in a cardiac cell, so that (a risk of) heart failure, is counteracted. In that case said vector preferably comprises a promoter suitable for expression in a cardiac cell. In another embodiment, however, a vector according to the invention comprises a ubiquitous promoter. It is advantageous to use an inducible promoter, so that expression of (at least one) nucleic acid of a vector according to the invention is regulated at will. Non-limiting examples of inducible promoters are cardiac troponin and alpha myosin heavy chain, which are cardiomyocyte specific promoters. In one embodiment a cardiac fibroblast specific promoter may be used.

A vector according to the invention is particularly suitable for therapy. Further provided is therefore a use of a vector as defined above for the preparation of a medicament and/or prophylactic agent for counteracting, treating, diminishing, delaying and/or preventing heart failure.

Also provided is a method for counteracting, treating, diminishing, delaying and/or preventing heart failure, in a subject, the method comprising administering a therapeutically amount of a vector according to the invention to the subject. As said before, said vector preferably comprises a retroviral, adenoviral, adeno-associated viral or lentiviral vector. In one embodiment, an AAV9 vector is used. An isolated or recombinant cell comprising a vector according to the invention is also herewith provided.

It is possible to provide cells with the pri-miRNA and/or pre-miRNA and/or miRNA *in vitro.* Such cells are particularly suitable for research purposes. Moreover, such cells are suitable for therapy. For instance, cells overexpressing one or more of miR-191, miR-30e, miR-744, miR-142-3p and solexa-2952-306 can be administered to a subject in order to decrease the risk of heart failure. Further provided is therefore a use of an isolated cell capable of overexpressing one or more of miR-191, miR-30e, miR-744, miR-142-3p and solexa-2952-306, or a functional part or derivative thereof, for the preparation of a medicament and/or prophylactic agent for counteracting, treating, diminishing, delaying and/or preventing heart failure. Alternatively, the use of an isolated cell capable of overexpressing, and preferably excreting, one or more of the proteins encoded by target mRNAs of the miRNAs indicated herein as being overexpressed in subjects suffering from heart failure is envisaged.

Pharmaceutical compositions allowing prevention and or treatment of heart failure comprising one or a combination of compounds indicated herein together with a pharmaceutically acceptable carrier, diluent or excipient are also provided herein.

A pharmaceutical composition according to the invention may comprise the pri-miRNA and/or pre-miRNA and/or miRNA, or a functional part or derivative thereof of a miRNA of which the downregulation or underexpression as indicated herein is associated with heart failure, and/or
- a compound capable of increasing or restoring the expression, amount and/or activity of said miRNA in a cell, and/or
- a compound capable of increasing interaction of the target mRNA with said miRNA, and/or
- a vector according to the invention, capable of expressing the said miRNA in a cell, and/or
- a cell capable of overexpressing said miRNA, or a functional part or derivative thereof, and/or
- a protein encoded by a target mRNA of a miRNA of which the upregulation or overexpression as indicated herein is associated with heart failure, or a vector or a cell capable of expressing said protein,
together with a pharmaceutically acceptable carrier, diluent or excipient.

Also provided is a method for counteracting, treating, diminishing, delaying and/or preventing heart failure, in a subject, the method comprising administering a therapeutically amount of a pharmaceutical composition according to the invention to the subject.

Further provided is a non-human animal comprising:
- an exogenous nucleic acid sequence comprising one or more of the pri-miRNA and/or pre-miRNA and/or miRNA, or a functional part or derivative thereof of which the up or downregulation is associated with heart failure as indicated herein, and/or
- an exogenous nucleic acid sequence encoding a compound capable of increasing or inhibiting the expression, amount and/or activity of miRNA, of which the up- or downregulation is associated with heart failure as indicated herein, or a functional part or derivative thereof, in a cell, and/or
- an exogenous nucleic acid sequence encoding a compound capable of increasing interaction of the target mRNA with the miRNA, of which the up- or downregulation is associated with heart failure as indicated herein.

Said non-human animal preferably comprises a mammal. In one preferred embodiment said non-human animal comprises a test animal such as a rodent, a rabbit, a goat, a cow, a sheep or a monkey. A non-human animal which has been provided with a vector, an isolated cell, and/or a pharmaceutical composition according to the invention is also provided. A non-human animal according to the invention is especially useful for screening, detection and/or identification of candidate compounds capable of inhibiting or decreasing expression, amount and/or activity of a miRNA that is uregulated in heart failure as indicated herein. Such non-human test animal is also especially useful for screening, detection and/or identification of candidate compounds capable of decreasing interaction of the target mRNA with its corresponding miRNA. Hence, a non-human test animal according to the invention is especially useful for screening, detection and/or identification of candidate compounds capable of inducing or enhancing heart failure. If a candidate compound appears to have this property, it is recommended to avoid administration to human subjects.

In methods of treatment according to the present invention, the subject is preferably a mammal, most preferably a human, and the miRNAs as indicated herein refer in preferred embodiments to the homo sapience (hsa) miRNAs.

In yet another embodiment, a method is provided for screening, detection and/or identification of candidate compounds capable of counteracting, treating, diminishing, delaying and/or preventing heart failure. In this embodiment, candidate compounds are typically screened for their potential capabilities of increasing the expression, amount and/or activity of a miRNA of which the downregulation is associated with heart failure, for their potential capabilities of decreasing the expression, amount and/or activity of a miRNA of which the upregulation is associated with heart failure, or which have capabilities of counteracting the effects of the aberrant protein expression associated with the aberrant miRNA expression as indicated herein. If a candidate compound appears to have this property, it is capable of counteracting or preventing heart failure. Further provided is therefore a method for determining whether a candidate compound is capable of counteracting, treating, diminishing, delaying and/or preventing heart failure, the method comprising:
- contacting said candidate compound with a cell, preferably with a cardiac cell, and
- determining whether said candidate compound is capable of counteracting in said cell the effects of the aberrant protein expression associated with the aberrant miRNA expression as indicated herein.

In one preferred embodiment, a candidate compound is contacted with a cell according to the present invention wherein expression of the miRNA (preferably which is downregulated in heart failure) is diminished, so that the effects of candidate compounds upon the miRNA expression or activity is more clearly visible. It is, however, also possible to use any cell or non-human animal currently known in the art in a screening method according to the invention. In one embodiment, a non-human animal according to the invention is used.

In another embodiment, candidate compounds are screened for their capability of increasing interaction of the target mRNA with the miRNAs as indicated herein, or with a functional part or derivative thereof. If a candidate compound appears to have this property in relation to the miRNA of which the downregulation is associated with heart disease, it is capable of counteracting or preventing heart failure. Further provided is therefore a method for determining whether a candidate compound is capable of counteracting, treating, diminishing, delaying and/or preventing heart failure, the method comprising:
- contacting said candidate compound with a cell, preferably with a cardiac cell, and
- determining whether said candidate compound is capable of increasing interaction of the mRNA with one or more of miR-191, miR-30e, miR-744, miR-142-3p and solexa-2952-306, or with a functional part or derivative thereof, within said cell.

In a preferred embodiment, a method for diagnosing a subject as suffering from heart failure or being at risk of developing heart failure according to the invention comprises (a) determining the level of at least one miRNA selected from miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, solexa-3927-221, miR-191, miR-302d, miR-30e, miR-744, miR-142-3p and/or solexa-2952-306, or precursor microRNAs (pre-miRNA) or microRNA primary transcripts thereof and (b) diagnosing whether the subject suffers from heart failure or is at risk of developing heart failure based on the level of said at least one genetic markers determined in step (a). A preferred miRNA is miR-423-5p.

In a further preferred embodiment, a method according to the invention comprises determining the level of at least two miRNAs selected from miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, solexa-3927-221, miR-191, miR-302d, miR-30e, miR-744, miR-142-3p and/or solexa-2952-306, or precursor microRNAs (pre-miRNA) or microRNA primary transcripts thereof, such as two miRNAs, three miRNAs, four miRNAs, five miRNAs, six miRNAs, seven miRNAs, eight miRNAs, nine miRNAs, ten miRNAs, fifteen miRNAs, twenty miRNAs, all miRNAs selected from miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, solexa-3927-221, miR-191, miR-302d, miR-30e, miR-744, miR-142-3p and/or solexa-2952-306, or precursor microRNAs (pre-miRNA) or microRNA primary transcripts thereof.

In a preferred embodiment, said at least two miRNAs are selected from the group consisting of miR-423-5p and miR-191*, miR-423-5p and miR-15b, miR-423-5p and miR-1228*, miR-423-5p and miR-181a, miR-423-5p and miR-18a*, miR-423-5p and miR-107, miR-423-5p and miR-299-3p, miR-423-5p and miR-342-3p, miR-423-5p and miR-652, miR-423-5p and miR-675, miR-423-5p and miR-129-5p, miR-423-5p and miR-18b*, miR-423-5p and miR-1254, miR-423-5p and miR-622, miR-423-5p and HS_202.1, miR-423-5p and miR-191, miR-423-5p and miR-302d, miR-423-5p and miR-30e, miR-423-5p and miR-744, miR-423-5p and miR-142-3p, miR-423-5p and solexa-2952-306, or miR-423-5p and solexa-3927-221.

A preferred method for diagnosing and/or prognosing according to the invention further comprises the detection of one or more biomarkers that may be used for diagnosing and/or prognosing heart failure. These biomarkers preferably comprise inflammation markers, such as C-reactive protein and inflammatory cytokines such as interleukin-6 and tumor necrosis factor (TNF); indicators for oxidative stress such as low-density lipoproteins, malondialdehyde and myeloperoxidase and isoprostane levels in plasma and urine; markers for remodelling of the extracellular-matrix of the ventricles such as propeptide procollagen type I and/or propeptide procollagen type III; neurohormones such as plasma levels of norepinephrine and endothelin-1; markers for myocyte injury such as cardiac troponins T and I; markers of cardiac remodeling and fibrosis such as galectin-3, and markers for myocyte stress such as natriuretic peptides BNP and N-terminal pro-brain natriuretic peptide (NT-pro-BNP), adrenomedullin and ST2, a member of the interleukin-1 receptor family. A further preferred method for diagnosing and/or prognosing according to the invention in addition comprises conventional methods such as, for example, electrocardiography, echocardiography, and/or cardiac magnetic resonance imaging (CMR).

In a further preferred embodiment, the results of a method for diagnosing and/or prognosing according to the invention determine at least in part a method of treatment of a subject suffering from heart failure or being at risk of developing heart failure. Possible treatment comprises dietary measures such as reduction of sodium intake, weight reduction in obese subjects, and/or discouraging of smoking; prescription of drugs such as angiotensin-converting enzyme inhibitors and/or beta-adrenoceptor antagonists; and/or invasive procedures such as revascularization procedures, mitral valve surgery, ventricular restoration and heart transplantation. The choice of a treatment may at least in part depend on the identity and/or the level of expression of at least one genetic marker selected from the group consisting of miR-423-5p, miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221, and precursor microRNAs (pre-miRNA) or microRNA primary transcripts thereof, that are determined in a sample obtained from a subject.

### Pharmaceutical Compositions and Therapeutic Uses

Pharmaceutical compositions can comprise polypeptides, polynucleotides or small molecules of the claimed invention, collectively called pharmaceutical compounds herein. The pharmaceutical compositions will comprise a therapeutically effective amount of either a biomarker protein, a polynucleotide or small molecule as described herein.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present invention, an effective dose will be from about 0.01 mg/ kg to 50 mg/kg or 0.05 mg/kg to about 10 mg/kg of the polynucleotide or polypeptide compositions in the individual to which it is administered.

A pharmaceutical composition can also contain a pharmaceutically acceptable carrier. The term "pharmaceutically acceptable carrier" refers to a carrier for administration of a therapeutic agent, such as a polypeptide, polynucleotide, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulphates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### Delivery Methods

Once formulated, the pharmaceutical compositions of the invention can be (1) administered directly to the subject; (2) delivered *ex vivo,* to cells derived from the subject; or (3) delivered in vitro for expression of recombinant proteins.

Direct delivery of the compositions will generally be accomplished by injection, either subcutaneously, intraperitoneally, intravenously or intramuscularly, or delivered to the interstitial space of a tissue. The compositions can also be administered into the nervous system. Other modes of administration include topical, oral, suppositories, and transdermal applications, needles, and particle guns or hyposprays. Dosage treatment may be a single dose schedule or a multiple dose schedule.

Methods for the *ex vivo* delivery and re-implantation of transformed cells into a subject are known in the art and described in e.g., International Publication No. WO 93/14778. Examples of cells useful in *ex vivo* applications include, for example, stem cells, particularly hematopoietic, lymph cells, macrophages, dendritic cells, or tumor cells.

Generally, delivery of nucleic acids for both *ex vivo* and *in vitro* applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Various methods are used to administer the therapeutic composition directly to a specific site in the body. Receptor-mediated targeted delivery of therapeutic compositions containing an antisense polynucleotide, subgenomic polynucleotides, or antibodies to specific tissues is also used. Receptor-mediated DNA delivery techniques are described in, for example, Findeis et al., Trends in Biotechnol. (1993) 11:202-205; Wu et a/., J. Biol. Chem. (1994) 269:542-46.

Pharmaceutical compositions containing polynucleotides are preferably administered in a range of about 100 ng to about 200 mg of polynucleotides for local administration in a gene therapy protocol. Concentration ranges of about 500 ng to about 50 mg, about 1 µg to about 2 mg, about 5 µg to about 500 µg, and about 20 µg to about 100 µg of polynucleotides can also be used during a gene therapy protocol. Factors such as method of action and efficacy of transformation and expression are considerations which will affect the dosage required for ultimate efficacy of the polynucleotides. Where greater expression is desired over a larger area of tissue, larger amounts of polynucleotides or the same amounts re-administered in a successive protocol of administrations, or several administrations to different adjacent or close tissue portions of, for example, a nerve ending or synaps, may be required to affect a positive therapeutic outcome. In all cases, routine experimentation in clinical trials will determine specific ranges for optimal therapeutic effect. A more complete description of gene therapy vectors, especially retroviral vectors, is contained in WO 98/00542, which is expressly incorporated herein.

The invention will now be illustrated by way of the following, non limiting Examples.

### EXAMPLES

### Example 1. Use of miRNAs as circulating biomarkers for the detection of heart failure

Blood was drawn from 2 healthy individuals and 3 heart failure patients. Plasma was collected after centrifugation for 15 minutes at 1550 x g. Small RNA was isolated using PARIS kit for RNA isolation (Applied Biosystems), according to the manufactures protocol. MiRNA expression profiles was performed using the Illumina human miRNA Expression Profiling Panel (User card for bead chip cat#MI-501-1001, part#11297760 RevA). This was a pilot-array. Normalization was performed using Genespring software.

A total of 9 miRNAs were found to be significantly upregulated in the blood of heart failure patients, and 6 miRNA were down-regulated. (See table 1, and figures 1 and 2).

These differentially regulated miRNAs may be used as biomarkers for the detection of heart failure.

**Table 1. Differentially expressed miRNAs in plasma of heart failure patients.**

| *Upregulated in HF* | | |
|---|---|---|
| | **p-value** | **fold change** |
| hsa-miR-191* | 0.00031 | 1.241047 up |
| has-miR-15b | 0.05 | 1.61 up |
| hsa-miR-1228* | 0.029037 | 8.52983 up |
| hsa-miR-181a | 0.057393 | 4.049339 up |
| hsa-miR-18a* | 0.098554 | 5.809895 up |
| hsa-miR-107 | 0.03166 | 5.999936 up |
| hsa-miR-299-3p | 0.046533 | 6.359402 up |
| hsa-miR-342-3p | 0.019917 | 5.593522 up |
| hsa-miR-652 | 0.001642 | 2.926441 up |
| | | |

| *Downregulated in HF* | | |
|---|---|---|
| | **p-value** | **fold change** |
| hsa-miR-191 | 0.018187 | 8.150075 down |
| hsa-miR-302d | 0.018313 | 15.54471 down |
| hsa-miR-30e | 0.012626 | 0.567286 down |
| hsa-miR-744 | 0.036437 | 0.183446 down |
| solexa-2952-306 | 0.035421 | 6.306514 down |
| solexa-3927-221 | 0.008918 | 5.52349 down |

### Example 2. Use of miRNAs as circulating biomarkers for the detection of heart failure. Definitive array.

The above example was repeated for an overlapping set of miRNAs on a new array that was produced specifically for this new screening. Blood was drawn from 12 healthy individuals and 12 heart failure patients. Plasma was collected after centrifugation for 15 minutes at 1550 x g. Small RNA was isolated using mirVana PARIS kit for RNA isolation (Applied Biosystems), according to the manufactures protocol. MiRNA expression profiles was performed using the Illumina human miRNA Expression Profiling Panel. Normalization was performed using Genespring software.

The results of the array as shown in Figure 4 were confirmed by QPCR. One miRNA (solexa-3927-221) was found to be upregulated in this experiment whereas in the pilot array experiment of Figure 1 it appeared downregulated. The QPCR results confirmed that the MiRNA was in fact upregulated in heart failure patients. The remaining miRNAs were confirmed.

These differentially regulated miRNAs may be used as biomarkers for the detection of heart failure.

### Example 3. Use of miRNAs as circulating biomarkers for the detection of heart failure. Validation in an independent clinical study.

### Methods

Human plasma samples were obtained with informed consent under a general waiver by the Academic Medical Center institutional review board for the proper secondary use of human material. For the dyspnea registry, plasma samples were obtained as part of a multicenter effort involving 3 centers in The Netherlands. Experiments described were performed on samples obtained at the Academic Medical Center.

Subjects were classified as HF cases when they met the Framingham criteria for the diagnosis and if circulating NT-proBNP was above 1000 ng/L. Subjects were classified as non-HF cases if clinical diagnosis excluded HF and the circulating NT-proBNP was below the age-related cutoff points published by Januzzi et al. (Januzzi et al. 2006. Eur Heart J 27:330). In total, 50 of the 77 patients screened for the dyspnea registry fulfilled the criteria.

Thirty-nine healthy controls and 50 subjects of the dyspnea registry were studied. Of the 50 patients, 20 subjects were diagnosed not to have HF and 30 subjects were diagnosed to have HF. MiRNA expression levels between cases and controls were compared using the students t-test or the Mann-Whitney-U-test. Analyses were conducted using logistic regression analysis. Receiver-operator-characteristics (ROC) curves were made across various cut-off levels of the predicted probabilities of the logistic regression model in differentiating HFcases from nonHF-cases or healthy controls. The area under the ROC curve (AUC) was estimated to assess the diagnostic accuracy of miRNAs. All analyses were performed using SPSS and all statistical tests were two-sided. For all analyses, p-values<0.05 were considered statistically significant.

### Myocardial tissue

We obtained post-mortem myocardial samples from subjects who had died due to idiopathic DCM and myocardial samples from subject who had died due to non-cardiac causes. Total RNA was extracted using Trizol (Invitrogen). RNA quality was assured and subsequent realtime PCR was used to assess expression levels of miR-423-5p. This was normalized for expression of U6 to evaluate the relative myocardial expression of miR-423-5p.

### Blood processing

Blood of all subjects was collected via a direct venous puncture into 2.7 ml sodium citrate containing tubes (BD Biosciences 363048). From the subjects for the miRNA array 10.8 ml blood was drawn and for real-time PCR we collected 5.4 ml blood. All blood was processed for isolation of plasma within 4 hours of collection. Blood was processed by spinning at 1550 x g for 10 minutes at room temperature. Plasma was carefully transferred to a fresh RNAse/DNAse free tube and stored at-80°C.

### RNA isolation

For the miRNA array we isolated RNA of 4.4 ml plasma and for the real-time PCR we isolated RNA of 500 µl plasma. Plasma was thawed on ice and RNA was isolated using the mirVana PARIS kit (Ambion) according to the manufacturer's protocol for liquid samples. The protocol was modified such that samples were extracted twice with an equal volume of acidphenol chloroform and the column was dried for 3 minutes after the last washing step and before elution.

### miRNA array

From the eluted samples for the miRNA array 50 µl was concentrated to 12 µl and for each sample 5 µl was used for the array. The Illumina human v2 miRNA expression profiling was performed on these samples. The Illumina miRNA beadchip probes 1146 miRNAs and other small RNAs predicted to be miRNAs.1,2 Raw data were pre-processed, summarized, logtransformed, and quantile normalized using the beadarray package (version 1.12.1) in the statistical software package R (version 2.9.0). Differential expression was assessed using a moderated t-test using the limma package (version 2.18.3). MicroRNAs were considered significantly differentially expressed if the P-values were less than 0.05.

### Real-Time PCR

For plasma samples a fixed volume of 8 µl of the eluate from the RNA isolation and for myocardial tissue samples 1 µg of input RNA was used as input in the reverse transcription reaction. Input RNA was reverse transcribed using the miScript reverse transcription kit (Qiagen) according to the manufacturer's protocol. The real-time PCR was performed using High Resolution Melting Master (Roche). MgCl2 was used in an end-concentration of 2.5 mmol/L and 2 µl of 8 times diluted cDNA was used in a total volume of 10 µl. The forward primers had the same sequence as the mature miRNA sequence with all U's changed into T's and the sequence of the reverse primer was GAATCGAGCACCAGTTACGC, which is complementary to the adapter sequence of the RTprimer (part of the miScript reverse transcription kit) used to create cDNA of miRNAs. Real- Time PCR reactions were performed on a LightCycler480 system II (Roche) using the following program: 10 minutes pre-incubation at 95°C and 40 cycli of 45 seconds of denaturation at 95°C, 45 seconds of annealing at 55°C, and 45 seconds of elongation at 72°C. Data were analyzed using LinRegPCR quantitative PCR data analysis software, version 11.3.3 The starting concentration of miRNAs estimated by this software were corrected for the estimated starting concentration of miR-1249, an endogenous miRNA stably expressed in our miRNA array.

### Results

### Expression Profiles of MiRNAs in Plasma of HF Patients

MiRNA arrays (Illumina beadchip, human v2 miRNA panel) were performed on RNA from plasma of 12 HF patients and 12 healthy controls. A total of 108 miRNAs were significantly differentially expressed between HF patients and controls. From these, we selected 16 miRNAs based on their fold changes and probability values for further validation.

### Validation of Candidate MiRNAs in an Independent Population

We validated the expression of 16 candidate miRNAs in three novel groups of subjects. The first group consisted of subjects from the dyspnea registry who were diagnosed with HF (HF cases, n=30), the second group of subjects were also obtained from the dyspnea registry but clinical diagnostics established them to be free of HF (non-HF cases, n=20), and the third group consisted of healthy controls (n=39). In HF cases, 19 of 30 subjects had an ejection fraction (EF) lower than 45%, whereas in non-HF cases 3 of 20 subjects had an EF lower than 45%. These latter three subjects therefore had left ventricular dysfunction, but lacked the clinical and NTproBNP criteria to diagnose HF. The expression level of the miRNAs was assessed by real-time PCR, and normalized by expression levels of miR-1249, a miRNA that was found to be unchanged in the arrays. The fold changes of miRNA levels for the HF cases versus healthy controls are shown in Figure 5.

### Diagnostic Accuracy of Candidate MiRNAs

One miRNA, miR-423-5p, was found to be a significant predictor of HF diagnosis in a multivariate logistic regression model including age and sex. Figure 6 shows that miR-423-5p is specifically increased in HF cases compared to both healthy controls and to dyspneic non-HF cases. MiR-423-5p distinguished HF cases from healthy controls with an area under the curve (AUC) of 0.91 (95% confidence interval, 0.84 to 0.98). The predictive power of miR-423-5p was also high within the dyspnea registry, when comparing HF and non-HF cases (AUC, 0.83; 95% confidence interval, 0.71 to 0.94). Circulating miR-423-5p correlated with NT-proBNP and EF (Spearman correlation coefficient: 0.43, probability value: 0.002; Spearman correlation coefficient: 0.34, probability value: 0.023, respectively). Expression of miR-423-5p was increased three-fold in failing human myocardium as compared to normal human hearts (see Figure 7).

Besides miR-423-5p, six other miRNAs (miR-18b*, miR-129-5p, miR-1254, miR-675, HS_202.1 and miR-622) were found to be increased in HF cases, of which miR-18b* and miR-675 are depicted in Figure 6. However, within the dyspnea population, miR-18b* is also slightly elevated in non-HF cases, whereas miR-675 is even upregulated in non-HF cases to the same level as HF cases. This exemplifies that some miRNAs initially identified by comparing HF cases to healthy controls actually appear also to be elevated when dyspnea is not caused by HF and therefore are less specific for HF. However, it is known that also NT-proBNP can be slightly increased with pulmonary disease attributable to right ventricular overload so that miRNAs like miR-18b* may still be valuable biomarkers of HF.

To investigate whether candidate miRNAs relate to disease severity or etiology, we categorized HF patients according to their EF, NYHA class or underlying etiology. Levels of circulating miR-423-5p and miR-18b* were higher in subjects with EF of ≤ 45%, compared to subjects with EF of >45%, but this did not reach statistical significance. Circulating levels of miR-423-5p, and also other miRNAs like miR-18b* increased with increasing NYHA class. Finally, some candidate miRNAs (miR-423-5p and miR-675, but not miR-18b*) were higher in atherosclerotic forms of HF as compared to nonatherosclerotic forms of HF (see Figure 8).

## Claims

1. A method for diagnosing a subject as suffering from heart failure or being at risk of developing heart failure, the method comprising:
a) determining the level of at least one genetic marker in a body fluid obtained from said subject, wherein said at least one genetic marker is a miRNA selected from the group consisting of miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221, and precursor microRNAs (pre-miRNA) or microRNA primary transcripts thereof;
b) diagnosing whether the subject suffers from heart failure or is at risk of developing heart failure based on the level of said at least one genetic marker determined in step a).

2. Method according to claim 1, wherein said subject is diagnosed as suffering from heart failure or prognosed as being at risk of developing heart failure when:
- the expression, amount, and/or activity of miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d and/or solexa-3927-221, or precursor microRNAs (pre-miRNA) or microRNA primary transcripts thereof, is upregulated relative to a control subject not suffering from or not being at risk of developing heart failure; and/or
- the expression, amount, and/or activity of miR-191, miR-30e, miR-744, miR-142-3p and/or solexa-2952-306, or precursor microRNAs (pre-miRNA) or microRNA primary transcripts thereof, is downregulated relative to a control subject not suffering from or not being at risk of developing heart failure.

3. A method for treating or preventing heart failure in a subject, the method comprising
diagnosing whether the subject suffers from heart failure or is at risk of developing heart failure based on the level of at least one genetic marker according to claim 1 or claim 2; and
treating the subject when the subject suffers from heart failure or is at risk of developing heart failure.

4. The method according to claim 3, comprising the step of:
- decreasing within said subject the expression, amount, and/or activity of at least one miRNA; or
- decreasing within said subject the interaction of at least one miRNA with its mRNA target; or
- increasing within said subject the expression, amount, and/or activity of the mRNA target of at least one miRNA,
wherein said at least one miRNA is selected from the group consisting of miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-302d and solexa-3927-221.

5. The method for treating or preventing heart failure in a subject the method according to claim 3, comprising the step of:
- increasing within said subject the expression, amount, and/or activity of at least one miRNA; or
- increasing within said subject the interaction of at least one miRNA with its mRNA target; or
- decreasing within said subject the expression, amount, and/or activity of the mRNA target of at least one miRNA,
wherein said at least one miRNA is selected from the group consisting of miR-191, miR-30e, miR-744, miR-142-3p and solexa-2952-306.

6. A method according to claim 5, wherein said step of increasing the expression, amount and/or activity or the interaction of said miRNA comprises administering to said subject said miRNA as functional miRNA, as precursor microRNA (pre-miRNA), or as microRNA primary transcript (pri-miRNA).

7. A miRNA selected from the group consisting of miR-191, miR-30e, miR-744, miR-142-3p and solexa-2952-306, or a precursor microRNA (pre-miRNA) or a microRNA primary transcript thereof, for use as a medicament, preferably for use in the treatment or prevention of heart failure.

8. A nucleic acid, capable of specifically binding to and thereby inhibiting the activity and/or function of at least one miRNA selected from the group consisting of miR-191*, miR-15b, miR-1228*, miR-181a, miR-18a*, miR-107, miR 299 3p, miR-342-3p, miR-652, miR-675, miR-129-5p, miR-18b*, miR-1254, miR-622, HS_202.1, miR-302d and solexa-3927-221, and precursor microRNAs (pre-miRNA) or microRNA primary transcripts thereof for use as a medicament, preferably for use in the treatment or prevention of heart failure.

9. A vector comprising a nucleic acid sequence encoding a miRNA or a precursor microRNA (pre-miRNA) or a microRNA primary transcript thereof according to claim 7 or a nucleic acid according to claim 8, and capable of expressing said (pre or pri) miRNA or nucleic acid, for use as a medicament, preferably for use in the treatment or prevention of heart failure.

10. A cell comprising a vector according to claim 9, for use as a medicament, preferably for use in the treatment or prevention of heart failure.

11. A pharmaceutical composition for treating or preventing heart failure in a subject comprising:
- a (pre- or pri-) miRNA according to claim 7, a nucleic acid according to claim 8, a vector according to claim 9, or a cell according to claim 10, and
- a pharmaceutically acceptable carrier, diluent or excipient.

12. A kit of parts for diagnosing a subject as suffering from heart failure or being at risk of developing heart failure comprising:
- at least one compound capable of specifically binding to at least one miRNA selected from the group consisting of miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, and solexa-3927-221, and precursor microRNAs (pre-miRNA) or microRNA primary transcripts thereof; and
- instructions for use of said compound for diagnosing a subject as suffering from heart failure or being at risk of developing heart failure according to a method of claim 1 or 2.

13. A method for determining whether a candidate compound is capable of treating or preventing heart failure in a human subject, the method comprising:
- contacting said candidate compound with a human tissue cell, preferably a cardiac tissue cell, and
- determining whether said candidate compound is capable of modulating the expression, amount and/or activity of miR-191*, miR-15b, miR-1228*, miR-181a, miR-18a*, miR-107, miR-299-3p, miR-342-3p, miR-652, miR-675, miR-129-5p, miR-18b*, miR-1254, miR-622, HS_202.1, miR-191, miR-302d, miR-30e, miR-744, miR-142-3p, solexa-2952-306, or solexa-3927-221 in said tissue cell.
